# FASCICULE DE BREVET EUROPEEN

(11) **EP 1 000 607 B1**
(45) Date de publication et mention de la délivrance du brevet: **06.02.2002**
(21) Numéro de dépôt: 99402740.7
(22) Date de dépôt: 04.11.1999
(51) Int. Cl.: A61K 7/02, A61K 7/043, A61K 7/032, A61K 7/48, A61K 7/00

(54) **Procédé de maquillage des matières kératiniques**
Schminkverfahren für Keratinfasern
Make-up method for keratin fibers

(30) Priorité: 06.11.1998 FR 9814110
(43) Date de publication de la demande: 17.05.2000
(73) Titulaire: L'OREAL, 75008 Paris (FR)
(72) Inventeur: Ramin, Roland, 75014 Paris (FR); Brenne, Ingrid, 94240 L'Hay-les-Roses (FR)
(74) Mandataire: Kromer, Christophe

(56) Documents cités:
- EP-A- 0 288 012
- EP-A- 0 764 436
- WO-A-98/31329
- GB-A- 2 238 242

## Description

La présente invention se rapporte à un procédé de maquillage des matières kératiniques comprenant l'application sur les matières kératiniques d'une première couche d'une composition filmogène et d'une deuxième couche de particules solides. L'invention a aussi pour objet un kit de maquillage pour la mise en oeuvre du procédé. Le procédé selon l'invention peut être appliqué sur la peau aussi bien du visage que du corps, sur les lèvres et sur les phanères comme les ongles, les cils, les sourcils ou les cheveux ; ce procédé est particulièrement approprié pour le maquillage des ongles.

La composition filmogène peut être un vernis-à-ongles, un fard à joue ou à paupières, un fond de teint, un produit de maquillage pour les lèvres ou un produit de maquillage du corps.

Les compositions de vernis à ongles comprennent généralement un polymère filmogène soit solubilisé dans un solvant organique, soit dispersé sous forme de particules dans un milieu aqueux. De tels vernis sont par exemple décrits dans les documents US-A-4158053 et FR-A-2578741.

Ces vernis à ongles, après l'application sur l'ongle d'une ou de plusieurs couches de la composition et après séchage, conduisent généralement à la formation d'un film lisse, continu et homogène, brillant ou mat. Certains films présentent aussi de bonnes propriétés cosmétiques telles qu'une bonne tenue et en particulier une bonne adhérence sur l'ongle, une bonne résistance à l'eau, aux frottements et aux chocs.

Avec l'évolution de la mode, les consommateurs, plus exigeants, recherchent de nouveaux produits de maquillage permettant d'obtenir des effets de maquillage originaux ou particuliers. Un besoin subsiste donc de disposer de produit de maquillage dont l'application sur un support comme la peau ou les phanères d'êtres humains permet d'obtenir un effet de maquillage différent de ceux des films continus et homogènes actuellement obtenus avec les produits disponibles sur le marché.

La présente invention a donc pour but de proposer une composition de maquillage permettant d'obtenir un maquillage en relief sur la peau et sur les phanères, tout en présentant une bonne tenue dans le temps.

La demanderesse a constaté qu'un nouveau type de maquillage de la peau et/ou des phanères pouvait être obtenu à l'aide d'un procédé particulier d'application d'une composition filmogène et de particules solides. Ce procédé permet de répartir et maintenir en surface des particules de manière plus ou moins régulière en surface : on obtient ainsi un maquillage en relief original, conférant du volume, du gonflant, aux matières kératiniques, par exemple aux ongles. En outre, le maquillage présente une bonne adhésion sur le support maquillé et une bonne tenue, notamment aux frottements et aux chocs. De plus, les particules donnent l'impression de la présence de bulles à la surface du maquillage.

De façon plus précise, l'invention a pour objet un procédé de maquillage des matières kératiniques consistant à appliquer sur les matières kératiniques une première couche d'une première composition filmogène comprenant un milieu cosmétiquement acceptable et au moins un premier polymère filmogène, puis à appliquer sur au moins une partie de la première couche, et avant le séchage de la première couché, une deuxième couche constituée essentiellement de particules solides insolubles dans le milieu cosmétiquement acceptable de la première composition et ayant une densité allant de 800 kg/m³ à 10000 kg/m³, les particules solides ayant une forme sensiblement sphérique ou ovoïdale.

L'invention a encore pour objet un kit de maquillage comprenant une première composition comprenant au moins un premier polymère filmogène dans un milieu cosmétiquement acceptable, et des particules solides ayant une forme sensiblement sphérique ou ovoïdale et ayant une densité allant de 800 kg/m³ à 10000 kg/m³.

Selon l'invention, la seconde couche est appliquée au moins sur une partie de la première couche, voire sur la totalité de la première couche, avant séchage de la première couche. Elle peut être appliquée soit à l'une des extrémités de la première couche, soit au milieu, ou encore de façon discontinue notamment sous forme de motifs géométriques, symétriques ou dissymétriques (par exemple sous forme de points, carrés, ronds, étoiles), répartis de façon aléatoire ou ordonnée, à contours précis ou flous.

Dans le procédé selon l'invention, la deuxième couche est obtenue par application directe des particules solides sans que celles-ci ne soient mélangées avec un milieu liquide, pour permettre une bonne répartition sur la surface de la première couche.

Selon un premier mode de réalisation du procédé de l'invention, la seconde couche peut être appliquée par saupoudrage des particules solides sur la première couche avant séchage. Les particules viennent alors au contact de la première couche et se répartissent de façon plus ou moins régulière sur toute la surface de la première couche. Les particules en excès qui ne sont pas au contact de la première couche ne se fixent pas et sont éliminées lorsque le support ainsi maquillé est secoué ou bien encore lorsque l'excédent de particules est éliminé à l'aide d'un pinceau, d'une brosse ou d'air pulsé. Le saupoudrage peut avantageusement être réalisé à travers un tamis ou un pochoir.

Selon un deuxième mode de réalisation du procédé selon l'invention, la surface de la première couche appliquée sur les matières kératiniques est mise en contact, avant séchage, directement avec les particules solides stockées, par exemple, dans un récipient. Dans le cas d'un vernis à ongles, on peut plonger directement les doigts dans un bain de bille.

Le procédé selon l'invention permet donc de déposer et de fixer une couche de particules solides bien homogène et d'épaisseur régulière conférant ainsi un maquillage original en relief. De plus, les particules de forme sensiblement sphérique ou ovoïdale confèrent un toucher doux au maquillage.

Cette architecture bicouche peut être adaptée pour tous les produits de maquillage de la peau aussi bien du visage que du scalp et du corps, des muqueuses comme les lèvres, et des phanères comme les ongles. Cette architecture peut aussi être appliquée sur les accessoires de maquillage comme les faux ongles, faux cils, perruques ou encore sur des pastilles ou des patchs adhérents sur la peau ou les lèvres (du type mouches).

Il est possible d'appliquer sur la deuxième couche de particules solides une troisième couche d'une deuxième composition filmogène comprenant dans un milieu cosmétiquément acceptable un deuxième polymère filmogène. Cette troisième couche permet notamment d'assurer une très bonne tenue de la deuxième couche sur la première couche ; elle permet aussi d'apporter des nuances de couleur, notamment lorsque la couleur de la troisième couche est différente de la couleur de la première couche et/ou de la couleur des particules de la deuxième couche.

L'invention se rapporte aussi à un support maquillé dont le maquillage est susceptible d'être obtenu par le procédé selon l'invention.

L'invention a encore pour objet l'utilisation de la première composition et des particules solides telles que définies précédemment pour l'obtention d'un maquillage en relief en surface, de bonne tenue.

L'invention a également pour objet l'utilisation de particules solides telles que définies précédemment pour l'obtention d'un maquillage en relief en surface, de bonne tenue.

Les inventeurs ont trouvé de façon surprenante que l'application des particules solides conformément au procédé selon l'invention permet d'obtenir un maquillage en relief uniforme en surface présentant une bonne adhésion sur le support maquillé et une bonne tenue notamment aux frottements et aux chocs. En revanche, en ajoutant les particules solides dans une composition filmogène telle que définie précédemment, les particules ont tendance à sédimenter pendant le stockage de la composition et il est difficile d'obtenir une répartition uniforme de ces particules dans la composition filmogène. Il s'ensuit que l'application de ce mélange, par exemple à l'aide d'un pinceau, est rendue difficile du fait de l'hétérogénéité du mélange et cette application ne permet pas d'obtenir une répartition uniforme des particules déposées sur le support à traiter. Ainsi, le procédé selon l'invention permet d'obtenir un maquillage en relief homogène comportant une répartition uniforme des particules solides. Ce procédé permet de recouvrir, jusqu'à saturation, la première couche et de former une couche d'épaisseur constante de particules.

Les particules solides utilisées dans le procédé selon l'invention ont une densité allant de 800 kg/m³ à 10000 kg/m³ (0,8 à 10 g/cm³), de préférence de 1000 kg/m³ à 3000 kg/m³ (1 à 3 g/cm³), et mieux de 2300 à 3000 kg/m³ (2,3 à 3 g/cm³) pour permettre un bon contact avec la première couche avant séchage et obtenir ainsi une bonne tenue des particules après séchage de la première couche.

Avantageusement, les particules solides ont une forme sensiblement sphérique, pour permettre leur bonne répartition lors de leur application sur la première couche.

Les particules solides utilisées dans le procédé selon l'invention peuvent avoir une taille moyenne allant de 2,5 µm à 5 mm, et mieux de 50 µm à 2 mm. Plus les particules sont de petites tailles, plus la tenue des particules sur la première couche est meilleure ; ces particules permettent aussi d'effectuer des dessins plus fins et plus précis, notamment à l'aide d'un pochoir.

Les particules solides peuvent être en tout matériau satisfaisant les propriétés de densités définies précédemment. Par exemple, les particules solides peuvent être en un matériau choisi parmi le verre, l'oxyde de zirconium, le carbure de tungstène, les plastiques comme les polyuréthanes, les polyamides, le polytétrafluoroéthylène, le polypropylène, les métaux comme l'acier, le cuivre, le laiton, le chrome, le bois, le marbre, l'onyx, le jade, la nacre naturelle, les pierres précieuses (diamant, émeraude, rubis, saphir), l'améthyste, l'aigue-marine. On utilise de préférence des billes de verres telles que celles vendues sous la dénomination "SILIBEADS® " par la société SIGMUND LINDNER ; ces billes confèrent un brillant et un scintillement au maquillage.

Les particules solides, déformables ou non, peuvent être pleines ou creuses, incolores ou colorées, enrobées ou non.

Selon le procédé de l'invention, le milieu cosmétiquement acceptable des première et deuxième compositions peut comprendre; indépendamment l'une de l'autre, un milieu aqueux ou un milieu solvant organique.

Parmi les polymères filmogènes utilisables dans la composition pour le procédé de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).
Les polymères filmogènes de type radicalaires peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

On utilise de préférence des polymères filmogènes radicalaires anioniques, c'est-à-dire des monomères ayant au moins un monomère à groupement acide.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₂₀, de préférence en C₁-C₈, des (méth)acrylates d'aryle, en particulier d'aryle en C₆-C₁₀, des (méth)acrylates d'hydroxyalkyle, en particulier d'hydroxyalkyle en C₂-C₆.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide et le N-t-octyl acrylamide.

Les polymères filmogènes vinyliques peuvent également résulter de l'homopolymérisation ou de la copolymérisation d'au moins un monomère choisi parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme polymère filmogène acrylique en dispersion aqueuse utilisable selon l'invention, on peut citer ceux vendus sous les dénominations NEOCRYL XK-90® , NEOCRYL A-1070® , NEOCRYL A-1090® , NEOCRYL BT-62® , NEOCRYL A-1079® , NEOCRYL A-523® par la société ZENECA, DOW LATEX 432® par la société DOW CHEMICAL.

On peut ainsi citer, parmi les polycondensats utilisables comme polymère filmogène, les polyuréthannes anioniques, cationiques, non-ioniques ou amphotères, les polyuréthannes-acryliques, les poly-uréthannes-polyvinylpirrolidones, les polyester-polyuréthannes, les polyéther-polyuréthannes, les polyurées, les polyurée/polyuréthannes, et leurs mélanges.
Le polyuréthanne filmogène peut être, par exemple, un copolymère polyuréthanne, polyurée/uréthanne, ou polyurée, aliphatique, cycloaliphatique ou aromatique, comportant seule ou en mélange :
- au moins une séquence d'origine polyester aliphatique et/ou cycloaliphatique et/ou aromatique, et/ou,
- au moins une séquence siliconée, ramifiée ou non, par exemple polydiméthylsiloxane ou polyméthylphénylsiloxane, et/ou
- au moins une séquence comportant des groupes fluorés.

Comme polymère filmogène polyuréthane en dispersion aqueuse utilisable selon l'invention, on peut notamment citer les polyester-polyuréthanes vendus sous les dénominations "AVALURE UR-405® ", "AVALURE UR-410® ", "AVALURE UR-425® ", "SANCURE 20.60® " par la société GOODRICH et les polyétherpolyuréthanes vendus sous les dénominations "SANCURE 878® " par la société GOODRICH, "NEOREZ R 970® " par la société ICI.

Parmi les polycondensats filmogènes, on peut également citer les polyesters, les polyesters amides, les polyesters à chaîne grasse, les polyamides, et les résines époxyesters, les résines résultant de la condensation de formaldéhyde avec une arylsulfonamide, les résines aryl-sulfonamide époxy.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutariquè, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphta-lènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO₃M, avec M représentant un atome d'hydrogène, un ion ammonium NH₄⁺ ou un ion métallique, comme par exemple un ion Na⁺, Li⁺, K⁺, Mg²⁺, Ca²⁺, Cu²⁺, Fe²⁺, Fe³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en contre un groupement -SO₃M : l'acide sulfoisophtalique, l'acide sulfotéréphtalique, l'acide sulfophtalique, l'acide 4-sulfonaphtalène-2,7-dicarboxylique.
On préfère utiliser dans les compositions objet de l'invention des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques tels que la nitrocellulose, l'acétate de cellulose, l'acétobutyrate de cellulose, l'acétopropionate de cellulose, l'éthyl cellulose, et leurs mélanges.

Les premier et/ou second polymères filmogènes peuvent être solubilisés ou dispersés sous forme de particules dans le milieu cosmétiquement acceptable correspondant de chaque composition selon l'invention. Respectivement, le premier et le deuxième polymère filmogène peuvent être généralement présents en une teneur allant de 1 % à 70 % en poids, par rapport au poids total respectivement de la première et seconde composition, et mieux allant de 10 % à 40 % en poids.

Comme solvant organique utilisable dans l'invention, on peut citer :
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les composés cycliques aromatiques liquides à température ambiante tels que le toluène et le xylène ;
- les aldéhydes liquides à température ambiante tels que le benzaldéhyde, l'acétaldéhyde
- leurs mélanges.

Ces solvants conviennent plus particulièrement pour le maquillage des ongles : la composition constitue alors un vernis à ongles.

Dans chaque composition à milieu solvant organique, le solvant organique peut être présent en une teneur allant de 30 à 99 % en poids, par rapport au poids total de chaque composition, et de préférence de 60 % à 90 % en poids.

Lorsque les compositions pour la mise en oeuvre du procédé selon l'invention contiennent un milieu aqueux, ce dernier peut être constitué essentiellement d'eau ou bien encore d'un mélange hydroalcoolique et en particulier contenant des monoalcools inférieurs en C₁-C₅. La teneur en eau dans chaque composition à milieu aqueux peut aller de 30 à 99 % en poids, par rapport au poids total de chaque composition, et de préférence de 60 % à 90 % en poids.

Pour améliorer les propriétés filmogènes de la première et/ou de la deuxième composition du procédé selon l'invention, la première et la deuxième compositions peuvent comprendre un agent auxiliaire de filmification.

Un tel agent auxiliaire de filmification peut être choisi parmi tous les composés connus de l'homme du métier comme étant susceptibles de remplir la fonction recherchée, et être notamment choisi parmi les agents plastifiants.

En outre, lorsque l'une des compositions pour la mise en oeuvre du procédé selon l'invention comprend un polymère filmogène sous forme de particules dispersées dans le milieu de la composition, l'agent auxiliaire de filmification peut aussi être choisi parmi les agents de coalescence.

Chaque composition du procédé selon l'invention peut en outre comprendre tout additif connu de l'homme du métier comme étant susceptible d'être incorporé dans une telle composition, tels que les agents épaississants, les agents d'étalement, les agents mouillants, les agents dispersants, les anti-mousses, les conservateurs, les filtres UV, les colorants, les pigments, les actifs, les tensioactifs, les agents hydratants, les parfums, les neutralisants, les stabilisants, les antioxydants. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Chaque composition selon l'invention peut être préparée par l'homme du métier sur la base de ses connaissances générales et selon l'état de la technique.

Avantageusement, les premières et deuxièmes compositions du procédé selon l'invention sont conditionnées dans des compartiments ou récipients distincts, accompagnés de moyens d'application appropriés, identiques ou différents, tels que les pinceaux, les brosses, les plumes, les éponges.

L'invention est illustrée plus en détail dans les exemples suivants.

### Exemple 1 :

On a préparé un vernis à ongles ayant la composition suivante :
. Nitrocellulose 10,9 %
. Résine toluène sulfonamide formaldéhyde 10,7 % (Ketjenflex MS80 vendu par AKZO)
. Acétyl citrate de tributyle 6,5 %
. Toluène 31,0 %
. Acétate de butyle 22,6 %
. Acétate d'éthyle 9,3 %
. Alcool isopropylique 7,7 %
. Stéaralkonium hectorite 1,3%
. Acide citrique 0,1 %

On a appliqué sur les ongles une première couche de ce vernis à ongles, puis on a saupoudré sur cette couche avant séchage, des microbilles de verre vendues sous la dénomination "Silibeads" art. 4500 par la société SIGMUND (densité = 2500 kg/m³ ; taille des particules environ de 0,3 à 0,4 mm). Après le séchage de la première couche, les microbilles en contact sont fixées et réparties de façon homogène à la surface de l'ongle ; l'excédent de billes est éliminé en agitant les doigts. On dépose ainsi environ de 800 à 1000 billes par cm².

On obtient alors un maquillage en relief des ongles présentant une bonne tenue, notamment une bonne résistance à l'usure, et un toucher doux ; de plus, les billes réfléchissent la lumière et confèrent un effet scintillant, brillant.

Pour renforcer la tenue du maquillage, on peut appliquer sur les particules une troisième couche de finition ayant la composition suivante :
- nitrocellulose 15 g
- plastifiant 6 g
- Solvants (acétate de butyle, acétate d'éthyle) qsp 100 g

### Exemple 2 :

On a préparé un vernis à ongles ayant la composition suivante :
. dispersion de polymère acrylique (40% de matière sèche) 38 g
. dispersion de polyuréthanne (30% de matière sèche) 50 g
. acide silicique colloïdal (Aerosil MOX80) 0,7 g
. additifs (colorants, actifs) 1,3 g
. eau qsp 100 g

On a appliqué sur les ongles une première couche de ce vernis à ongles, puis on a saupoudré sur cette couche avant séchage, des billes d'acier ayant une densité = 7900 kg/m³ et une taille de particules d'environ 2 mm. Après le séchage de la première couche, les microbilles en contact sont fixées et réparties de façon homogène à la surface de l'ongle ; l'excédent de billes est éliminé en agitant les doigts.

On obtient alors un maquillage en relief des ongles présentant une bonne tenue, notamment une résistance à l'usure, et un toucher doux.

### Exemple 3 :

On a préparé un vernis à ongles ayant la composition suivante :
- polymères filmogènes (nitrocellulose, résine) 28 g
- plastifiant 7 g
- alcool isopropylique 5 g
- guar éthylé de degré de substitution d'environ 2,5 (1) 3 g
- pigments 1 g
- acétate de éthyle/acétate de butyle qsp 100 g

### (1) vendu sous la dénomination N-HANCE AG 200® par Aqualon

On a appliqué sur les ongles une première couche de ce vernis à ongles, puis on a saupoudré sur cette couche, avant séchage, de la poudre de nylon vendues sous la dénomination "ORGASOL D 2002 Nat Cos" par la société ATOCHEM Après le séchage de la première couche, les particules en contact sont fixées et réparties de façon homogène à la surface de l'ongle ; l'excédent de particules est éliminé en agitant les doigts.(densité = 1020 kg/m³ ; taille des particules environ de 20 µm).

On obtient ainsi un maquillage en relief présentant un toucher doux.

## Revendications

1. Procédé de maquillage des matières kératiniques comprenant l'application sur les matières kératiniques d'une première couche d'une première composition filmogène comprenant un milieu cosmétiquement acceptable et au moins un premier polymère filmogène, puis l'application sur au moins une partie de la première couche, et avant le séchage de la première couche, d'une deuxième couche constituée essentiellement de particules solides insolubles dans le milieu cosmétiquement acceptable de la première composition et ayant une densité allant de 800 kg/m³ à 10000 kg/m³, les particules solides ayant une forme sensiblement sphérique ou ovoïdale.

2. Procédé selon la revendication 1, **caractérisé par le fait que** les particules solides ont une densité allant de 1000 kg/m³ à 3000 kg/m³.

3. Procédé selon la revendication 1 ou 2, **caractérisé par le fait que** les particules solides ont une densité allant de 2300 kg/m³ à 3000 kg/m³.

4. Procédé selon l'une quelconque des revendications précédentes, **caractérisé par le fait que** les particules solides ont une taille allant de 2,5 µm à 5 mm, et mieux de 50 µm à 2 mm.

5. Procédé selon l'une quelconque des revendications précédentes, **caractérisé par le fait que** les particules solides ont une forme sensiblement sphérique.

6. Procédé selon l'une quelconque des revendications précédentes, **caractérisé par le fait que** les particules solides sont constituées en un matériau choisi dans le groupe formé par le verre, l'oxyde de zirconium, le carbure de tungstène, les polyuréthanes, les polyamides, le polytétrafluoroéthylène, le polypropylène, les métaux comme l'acier, le cuivre, le laiton, le chrome, le bois, le marbre, l'onyx, le jade, la nacre naturelle, les pierres précieuses, l'améthyste, l'aigue-marine.

7. Procédé selon l'une quelconque des revendications précédentes, **caractérisé par le fait que** les particules solides sont des microbilles de verre.

8. Procédé selon l'une quelconque des revendications précédentes, **caractérisé par le fait que** l'on applique sur la deuxième couche une troisième couche d'une deuxième composition filmogène comprenant un milieu cosmétiquement acceptable et au moins un deuxième polymère filmogène.

9. Procédé selon l'une quelconque des revendications précédentes, **caractérisé par le fait que** la première et et/ou la deuxième composition comprennent un milieu solvant.

10. Procédé selon l'une quelconque des revendications précédentes, **caractérisé par le fait que** le premier et/ou second polymère filmogène sont solubilisés ou dispersés sous forme de particules dans le milieu cosmétiquement acceptable correspondant.

11. Procédé selon l'une quelconque des revendications précédentes, **caractérisé par le fait que** les premier et deuxième polymères filmogènes sont choisis, indifféremment l'un de l'autre, dans le groupe formé par les polymères radicalaires, les polycondensats et les polymères d'origine naturelle.

12. Procédé selon l'une quelconque des revendications précédentes, **caractérisé par le fait que** les premier et deuxième polymères filmogènes sont choisis, indifféremment l'un de l'autre, dans le groupe formé par les polymères vinyliques, les polyuréthanes, les polyesters, les polymères cellulosiques.

13. Procédé selon l'une quelconque des revendications précédentes, **caractérisé par le fait que** la teneur en respectivement premier et second polymère filmogène va de 1 % à 70 % en poids, par rapport au poids total respectivement de la première et seconde composition, et de préférence de 10 % à 40 % en poids.

14. Procédé selon l'une quelconque des revendications précédentes, **caractérisé par le fait que** la première et/ou seconde composition comprennent en outre au moins un additif choisi dans le groupe formé par les agents auxiliaires de filmification, les agents épaississants, les agents d'étalement, les agents mouillants, les agents dispersants, les anti-mousses, les conservateurs, les filtres UV, les colorants, les pigments, les actifs, les tensioactifs, les agents hydratants, les parfums, les neutralisants, les stabilisants, les antioxydants.

15. Procédé selon la revendication 14, **caractérisé par le fait que** l'agent auxiliaire de filmification est présent en une teneur allant de 0,5 % à 20 % en poids, par rapport au poids total de chaque première et seconde composition, et de préférence de 2 à 10 % en poids.

16. Procédé selon l'une quelconque des revendications précédentes, **caractérisé par le fait que** la première et/ou seconde composition sont sous la forme de vernis à ongles, de fard à joue ou à paupières, de fond de teint, de produit de maquillage des lèvres ou du corps.

17. Procédé selon l'une quelconque des revendications précédentes, **caractérisé par le fait que** la deuxième couche de particules solides est déposée sur la première couche par saupoudrage des particules solides sur la première couche.

18. Procédé selon l'une quelconque des revendications 1 à 17, **caractérisé par le fait que** la deuxième couche de particules solides est déposée sur la première couche par contact de la première couche avec lesdites particules solides.

19. Kit de maquillage comprenant dans des récipients distincts :
- une première composition comprenant au moins un premier polymère filmogène dans un milieu cosmétiquement acceptable, et
- des particules solides ayant une forme sensiblement sphérique ou ovoïdale et ayant une densité allant de 800 kg/m³ à 10000 kg/m³.

20. Kit de maquillage selon la revendication 19, **caractérisé par le fait que** les particules solides ont une taille allant de 2,5 µm à 5 mm.

21. Kit de maquillage selon l'une des revendications 19 ou 20, **caractérisé par le fait que** les particules solides ont une forme sensiblement sphérique.

22. Kit de maquillage selon l'une quelconque des revendications 19 à 21, **caractérisé par le fait qu'**il comprend en outre une deuxième composition filmogène .

23. Kit de maquillage selon l'une quelconque des revendications 19 à 22, **caractérisé par le fait que** la première et/ou la deuxième composition comprennent un milieu solvant.

24. Kit de maquillage selon l'une quelconque des revendications 19 à 23, **caractérisé par le fait que** le premier et/ou second polymère filmogène sont solubilisés ou dispersés sous forme de particules dans le milieu cosmétiquement acceptable correspondant.

25. Kit de maquillage selon l'une quelconque des revendications 19 à 24, **caractérisé par le fait que** les premier et deuxième polymères filmogènes sont choisis, indifféremment l'un de l'autre, dans le groupe formé par les polymères radicalaires, les polycondensats et les polymères d'origine synthétique.

26. Kit de maquillage selon l'une quelconque des revendications 19 à 25, **caractérisé par le fait que** les premier et deuxième polymères filmogènes sont choisis, indifféremment l'un de l'autre, dans le groupe formé par les polymères vinyliques, les polyuréthanes, les polyesters, les polymères cellulosiques.

27. Kit de maquillage selon l'une quelconque des revendications 19 à 26, **caractérisé par le fait que** la teneur en respectivement premier et second polymère filmogène va de 1 % à 70 % en poids, par rapport au poids total respectivement de la première et seconde composition, et de préférence de 10 % à 40 % en poids.

28. Kit de maquillage selon l'une quelconque des revendications 19 à 27, **caractérisé par le fait que** la première et/ou seconde composition comprennent en outre au moins un additif choisi dans le groupe formé par les agents auxiliaires de filmification, les agents épaississants, les agents d'étalement, les agents mouillants, les agents dispersants, les anti-mousses, les conservateurs, les filtres UV, les colorants, les pigments, les actifs, les tensioactifs, les agents hydratants, les parfums, les neutralisants, les stabilisants, les antioxydants.

29. Kit de maquillage selon la revendication 28, **caractérisé par le fait que** l'agent auxiliaire de filmification est présent en une teneur allant de 0,5 % à 20 % en poids, par rapport au poids total de chaque première et/ou seconde composition, et de préférence de 2 à 10 % en poids.

30. Kit de maquillage selon l'une quelconque des revendications 19 à 29, **caractérisé par le fait que** la première et/ou seconde composition sont sous la forme de vernis à ongles, de fard à joue ou à paupières, de fond de teint, de produit de maquillage des lèvres ou du corps.

31. Kit de maquillage selon l'une quelconque des revendications 19 à 30, **caractérisé par le fait qu'**il contient des moyens pour appliquer sur les matières kératiniques la première et la seconde compositions.

32. Kit de maquillage selon la revendication 31, **caractérisé par le fait que** les moyens sont choisis dans le groupe formé par les pinceaux, les brosses, les plumes, les éponges.

33. Kit de maquillage selon l'une des revendications 19 à 32, **caractérisé par le fait que** les première et seconde compositions et les particules solides sont conditionnées dans des compartiments ou récipients distincts.

34. Support maquillé comprenant un maquillage susceptible d'être obtenu selon le procédé tel que défini selon l'une des revendications 1 à 18, le support étant choisi parmi les faux-ongles, les faux-cils, les perruques, les pastilles et les patchs adhérents sur la peau ou les lèvres.

35. Utilisation dans un produit de maquillage d'une première composition filmogène comprenant un milieu cosmétiquement acceptable et au moins un premier polymère filmogène, et de particules solides ayant une forme sensiblement sphérique ou ovoïdale et ayant une densité allant de 800 kg/m³ à 10000 kg/m³, pour l'obtention d'un maquillage en relief en surface, de bonne tenue.

36. Utilisation dans un produit de maquillage de particules solides ayant une forme sensiblement sphérique ou ovoïdale et ayant une densité allant de 800 kg/m³ à 10000 kg/m³, pour l'obtention d'un maquillage en relief en surface, de bonne tenue.

## Patentansprüche

1. Verfahren zum Schminken von Keratinsubstanzen, das umfaßt, auf die Keratinsubstanzen eine erste Schicht einer ersten filmbildenden Zusammensetzung aufzutragen, die ein kosmetisch akzeptables Medium und mindestens ein erstes filmbildendes Polymer enthält, und dann auf zumindest einem Teil der ersten Schicht vor dem Trocknen der ersten Schicht eine zweite Schicht aufzubringen, die im wesentlichen aus festen Partikeln besteht, die in dem kosmetisch akzeptablen Medium der ersten Zusammensetzung unlöslich sind und die eine Dichte von 800 bis 10000 kg/m³ aufweisen, wobei die festen Partikel im wesentlichen kugelförmig oder eiförmig sind.

2. Verfahren nach Anspruch 1, **dadurch gekennzeichnet, daß** die festen Partikel eine Dichte von 1000 bis 3000 kg/m³ aufweisen.

3. Verfahren nach Anspruch 1 oder 2, **dadurch gekennzeichnet, daß** die festen Partikel eine Dichte von 2300 bis 3000 kg/m³ aufweisen.

4. Verfahren nach einem der vorhergehenden Ansprüche, **dadurch gekennzeichnet, daß** die festen Partikel eine Größe von 2,5 µm bis 5 mm und besser 50 µm bis 2 mm aufweisen.

5. Verfahren nach einem der vorhergehenden Ansprüche, **dadurch gekennzeichnet, daß** die festen Partikel eine im wesentlichen sphärische Form aufweisen.

6. Verfahren nach einem der vorhergehenden Ansprüche, **dadurch gekennzeichnet, daß** die festen Partikel aus einem Material bestehen, das ausgewählt ist unter: Glas, Zirconiumoxid, Wolframcarbid, Polyurethanen, Polyamiden, Polytetrafluorethylen, Polypropylen, Metallen, wie Stahl, Kupfer, Messing und Chrom, Holz, Marmor, Onyx, Jade, Perlmutt, Edelsteinen, Amethyst und Aquamarin.

7. Verfahren nach einem der vorhergehenden Ansprüche, **dadurch gekennzeichnet, daß** es sich bei den festen Partikeln um Glaskugeln handelt.

8. Verfahren nach einem der vorhergehenden Ansprüche, **dadurch gekennzeichnet, daß** auf die zweite Schicht eine dritte Schicht einer zweiten filmbildenden Zusammensetzung aufgetragen wird, die ein kosmetisch akzeptables Medium und mindestens ein zweites filmbildendes Polymer enthält.

9. Verfahren nach einem der vorhergehenden Ansprüche, **dadurch gekennzeichnet, daß** die erste Zusammensetzung und/oder die zweite Zusammensetzung ein Lösungsmittelmedium enthält.

10. Verfahren nach einem der vorhergehenden Ansprüche, **dadurch gekennzeichnet, daß** das erste filmbildende Polymer und/oder das zweite filmbildende Polymer in dem entsprechenden kosmetisch akzeptablen Medium solubilisiert oder in Form von Partikeln dispergiert ist.

11. Verfahren nach einem der vorhergehenden Ansprüche, **dadurch gekennzeichnet, daß** die ersten und zweiten filmbildenden Polymere unabhängig voneinander unter den durch radikalische Polymerisation hergestellten Polymeren, den Polykondensaten und den Polymeren natürlicher Herkunft ausgewählt sind.

12. Verfahren nach einem der vorhergehenden Ansprüche, **dadurch gekennzeichnet, daß** die ersten und zweiten filmbildenden Polymere unabhängig voneinander unter den Vinylpolymeren, Polyurethanen, Polyestern und Cellulosepolymeren ausgewählt sind.

13. Verfahren nach einem der vorhergehenden Ansprüche, **dadurch gekennzeichnet, daß** der Mengenanteil des ersten filmbildenden Polymers bzw. des zweiten filmbildenden Polymers im Bereich von 1 bis 70 Gew.-%, bezogen auf das Gesamtgewicht der ersten bzw. zweiten Zusammensetzung, und vorzugsweise 10 bis 40 Gew.-% liegt.

14. Verfahren nach einem der vorhergehenden Ansprüche, **dadurch gekennzeichnet, daß** die erste und/oder zweite Zusammensetzung ferner mindestens einen Zusatzstoff enthält, der ausgewählt ist unter: zusätzlichen Filmbildnern, Verdickungsmitteln, Spreitmitteln, Netzmitteln, Dispergiermitteln, Antischaummitteln, Konservierungsmitteln, UV-Filtern, Farbmitteln, Pigmenten, Wirkstoffen, grenzflächenaktiven Stoffen, Hydratisierungsmitteln, Parfums, Neutralisationsmitteln, Stabilisierungsmitteln und Antioxidantien.

15. Verfahren nach Anspruch 14, **dadurch gekennzeichnet, daß** der zusätzliche Filmbildner in einem Mengenanteil im Bereich von 0,5 bis 20 Gew.-%, bezogen auf das Gesamtgewicht der ersten und zweiten Zusammensetzung, und vorzugsweise 2 bis 10 Gew.-% vorliegt.

16. Verfahren nach einem der vorhergehenden Ansprüche, **dadurch gekennzeichnet, daß** die erste und/oder zweite Zusammensetzung als Nagellack, Wangenrouge, Lidschatten, Make-up oder Produkt zum Schminken der Lippen oder des Körpers vorliegt.

17. Verfahren nach einem der vorhergehenden Ansprüche, **dadurch gekennzeichnet, daß** die zweite Schicht der festen Partikel auf der ersten Schicht aufgebracht wird, indem die erste Schicht mit den festen Partikeln bestreut wird.

18. Verfahren nach einem der Ansprüche 1 bis 17, **dadurch gekennzeichnet, daß** die zweite Schicht der festen Partikel auf der ersten Schicht aufgebracht wird, indem die erste Schicht mit den festen Partikeln in Kontakt gebracht wird.

19. Kit zum Schminken, der in verschiedenen Behältnissen enthält:
- eine erste Zusammensetzung, die in einem kosmetisch akzeptablen Medium mindestens ein erstes filmbildendes Polymer enthält, und
- feste Partikel, die im wesentlichen kugelförmig oder eiförmig geformt sind und eine Dichte von 800 bis 10000 kg/m³ aufweisen.

20. Kit zum Schminken nach Anspruch 19, **dadurch gekennzeichnet, daß** die festen Partikel eine Größe von 2,5 µm bis 5 mm aufweisen.

21. Kit zum Schminken nach einem Ansprüche 19 oder 20, **dadurch gekennzeichnet, daß** die festen Partikel eine im wesentlichen sphärische Form aufweisen.

22. Kit zum Schminken nach einem der Ansprüche 19 bis 21, **dadurch gekennzeichnet, daß** er ferner eine zweite filmbildende Zusammensetzung enthält.

23. Kit zum Schminken nach einem der Ansprüche 19 bis 22, **dadurch gekennzeichnet, daß** die erste Zusammensetzung und/oder die zweite Zusammensetzung ein Lösungsmittelmedium enthält.

24. Kit zum Schminken nach einem der Ansprüche 19 bis 23, **dadurch gekennzeichnet, daß** das erste filmbildende Polymer und/oder das zweite filmbildende Polymer in dem entsprechenden kosmetisch akzeptablen Medium solubilisiert oder in Form von Partikeln dispergiert ist.

25. Kit zum Schminken nach einem der Ansprüche 19 bis 24, **dadurch gekennzeichnet, daß** die ersten und zweiten filmbildenden Polymere unabhängig voneinander unter den durch radikalisch Polymerisation hergestellten Polymeren, den Polykondensaten und den Polymeren natürlicher Herkunft ausgewählt sind.

26. Kit zum Schminken nach einem der Ansprüche 19 bis 25, **dadurch gekennzeichnet, daß** die ersten und zweiten filmbildenden Polymere unabhängig voneinander unter den Vinylpolymeren, Polyurethanen, Polyestern und Cellulosepolymeren ausgewählt sind.

27. Kit zum Schminken nach einem der Ansprüche 19 bis 26, **dadurch gekennzeichnet, daß** der Mengenanteil des ersten filmbildenden Polymers bzw. des zweiten filmbildenden Polymers im Bereich von 1 bis 70 Gew.-%, bezogen auf das Gesamtgewicht der ersten bzw. zweiten Zusammensetzung, und vorzugsweise 10 bis 40 Gew.-% liegt.

28. Kit zum Schminken nach einem der Ansprüche 19 bis 27, **dadurch gekennzeichnet, daß** die erste und/oder zweite Zusammensetzung ferner mindestens einen Zusatzstoff enthält, der ausgewählt ist unter: zusätzlichen Filmbildnern, Verdickungsmitteln, Spreitmitteln, Netzmitteln, Dispergiermitteln, Antischaummitteln, Konservierungsmitteln, UV-Filtern, Farbmitteln, Pigmenten, Wirkstoffen, grenzflächenaktiven Stoffen, Hydratisierungsmitteln, Parfums, Neutralisationsmitteln, Stabilisierungsmitteln und Antioxidantien.

29. Kit zum Schminken nach Anspruch 28, **dadurch gekennzeichnet, daß** der zusätzliche Filmbildner in einem Mengenanteil im Bereich von 0,5 bis 20 Gew.-%, bezogen auf das Gesamtgewicht der ersten und/oder zweiten Zusammensetzung, und vorzugsweise 2 bis 10 Gew.-% vorliegt.

30. Kit zum Schminken nach einem der Ansprüche 19 bis 29, **dadurch gekennzeichnet, daß** die erste und/oder zweite Zusammensetzung als Nagellack, Wangenrouge, Lidschatten, Make-up oder Produkt zum Schminken der Lippen oder des Körpers vorliegt.

31. Kit zum Schminken nach einem der Ansprüche 19 bis 30, **dadurch gekennzeichnet, daß** er Mittel enthält, um die erste und zweite Zusammensetzung auf die Keratinsubstanzen aufzutragen.

32. Kit zum Schminken nach Anspruch 31, **dadurch gekennzeichnet, daß** die Mittel unter den Pinseln, Bürsten, Federn und Schwämmen ausgewählt sind.

33. Kit zum Schminken nach einem der Ansprüche 19 bis 31, **dadurch gekennzeichnet, daß** die erste Zusammensetzung, die zweite Zusammensetzung und die festen Partikel in unterschiedlichen Abteilungen oder Behältnissen konfektioniert sind.

34. Geschminkter Träger, der eine Schminke aufweist, die nach dme Verfahren nach einem der Ansprüche 1 bis 18 hergestellt werden kann, wobei der Träger unter den künstlichen Nägeln, falschen Wimpern, Perücken, Plättchen oder Patches, die auf der Haut oder den Lippen haften, ausgewählt ist.

35. Verwendung einer ersten filmbildenden Zusammensetzung, die ein kosmetisch akzeptables Medium und mindestens ein erstes filmbildendes Polymer enthält, und fester Partikel, die im wesentlichen kugelförmig oder eiförmig geformt sind und eine Dichte von 800 bis 10000 kg/m³ aufweisen, in einem Produkt zum Schminken, um eine Schminke mit Oberflächenrelief und guter Haftung herzustellen.

36. Verwendung von festen Partikeln, die im wesentlichen kugelförmig oder eiförmig geformt sind und eine Dichte von 800 bis 10000 kg/m³ aufweisen, in einem Produkt zum Schminken, um eine Schminke mit Oberflächenrelief und guter Haftung herzustellen.

## Claims

1. Process for making up keratin substances, which consists in applying to the keratin substances a first coat of a first film-forming composition comprising a cosmetically acceptable medium and at least one first film-forming polymer, followed by applying to at least a part of the first coat, and before the first coat has dried, a second coat consisting essentially of solid particles that are insoluble in the cosmetically acceptable medium of the first composition and having a density ranging from 800 kg/m³ to 10,000 kg/m³, the solid particles having a substantially spherical or ovoid shape.

2. Process according to Claim 1, **characterized in that** the solid particles have a density ranging from 1000 kg/m³ to 3000 kg/m³.

3. Process according to Claim 1 or 2, **characterized in that** the solid particles have a density ranging from 2300 kg/m³ to 3000 kg/m³.

4. Process according to any one of the preceding claims, **characterized in that** the solid particles have a size ranging from 2.5 µm to 5 mm and better still from 50 µm to 2 mm.

5. Process according to any one of the preceding claims, **characterized in that** the solid particles have a substantially spherical shape.

6. Process according to any one of the preceding claims, **characterized in that** the solid particles consist of a material chosen from the group formed by glass, zirconium oxide, tungsten carbide, polyurethanes, polyamides, polytetrafluoroethylene, polypropylene, metals such as steel, copper, brass or chromium, wood, marble, onyx, jade, natural mother-of-pearl, precious stones, amethyst or aquamarine.

7. Process according to any one of the preceding claims, **characterized in that** the solid particles are glass microbeads.

8. Process according to any one of the preceding claims, **characterized in that** a third coat of a second film-forming composition comprising a cosmetically acceptable medium and at least one second film-forming polymer is applied to the second coat.

9. Process according to any one of the preceding claims, **characterized in that** the first and/or the second composition comprise a solvent medium.

10. Process according to any one of the preceding claims, **characterized in that** the first and/or second film-forming polymer are dissolved or dispersed in the form of particles in the corresponding cosmetically acceptable medium.

11. Process according to any one of the preceding claims, **characterized in that** the first and second film-forming polymers are chosen, independently of each other, from the group formed by radical-mediated polymers, polycondensates and polymers of natural origin.

12. Process according to any one of the preceding claims, **characterized in that** the first and second film-forming polymers are chosen, independently of each other, from the group formed by vinyl polymers, polyurethanes, polyesters and cellulose polymers.

13. Process according to any one of the preceding claims, **characterized in that** the content of first and second film-forming polymer, respectively, ranges from 1% to 70% by weight, relative to the total weight of the first and second composition, respectively, and preferably from 10% to 40% by weight.

14. Process according to any one of the preceding claims, **characterized in that** the first and/or second composition also comprise at least one additive chosen from the group formed by additional film-forming agents, thickeners, spreading agents, wetting agents, dispersing agents, anti-foaming agents, preserving agents, UV screening agents, dyes, pigments, active agents, surfactants, moisturizers, fragrances, neutralizing agents, stabilizers and antioxidants.

15. Process according to Claim 14, **characterized in that** the additional film-forming agent is present in a content ranging from 0.5% to 20% by weight, relative to the total weight of each first and second composition, and preferably from 2 to 10% by weight.

16. Process according to any one of the preceding claims, **characterized in that** the first and/or second composition are in the form of a nail varnish, a face powder, an eyeshadow, a foundation or a make-up product for the lips or the body.

17. Process according to any one of the preceding claims, **characterized in that** the second coat of solid particles is deposited on the first coat by sprinkling the solid particles onto the first coat.

18. Process according to any one of Claims 1 to 17, **characterized in that** the second coat of solid particles is deposited on the first coat by contact of the first coat with the said solid particles.

19. Make-up kit comprising in separate containers:
- a first composition comprising at least one first film-forming polymer in a cosmetically acceptable medium, and
- solid particles having a substantially spherical or ovoid shape and having a density ranging from 800 kg/m³ to 10,000 kg/m³.

20. Make-up kit according to Claim 19, **characterized in that** the solid particles have a size ranging from 2.5 µm to 5 mm.

21. Make-up kit according to either of Claims 19 and 20, **characterized in that** the solid particles have a substantially spherical shape.

22. Make-up kit according to any one of Claims 19 to 21, **characterized in that** it also comprises a second film-forming composition.

23. Make-up kit according to any one of Claims 19 to 22, **characterized in that** the first and/or the second composition comprise a solvent medium.

24. Make-up kit according to any one of Claims 19 to 23, **characterized in that** the first and/or second film-forming polymer are dissolved or dispersed in the form of particles in the corresponding cosmetically acceptable medium.

25. Make-up kit according to any one of Claims 19 to 24, **characterized in that** the first and second film-forming polymers are chosen, independently of each other, from the group formed by radical-mediated polymers, polycondensates and polymers of synthetic origin.

26. Make-up kit according to any one of Claims 19 to 25, **characterized in that** the first and second film-forming polymers are chosen, independently of each other, from the group formed by vinyl polymers, polyurethanes, polyesters and cellulose polymers.

27. Make-up kit according to any one of Claims 19 to 26, **characterized in that** the content of first and second film-forming polymer, respectively, ranges from 1% to 70% by weight, relative to the total weight of the first and second composition, respectively, and preferably from 10% to 40% by weight.

28. Make-up kit according to any one of Claims 19 to 27, **characterized in that** the first and/or second composition also comprise at least one additive chosen from the group formed by additional film-forming agents, thickeners, spreading agents, wetting agents, dispersing agents, anti-foaming agents, preserving agents, UV screening agents, dyes, pigments, active agents, surfactants, moisturizers, fragrances, neutralizing agents, stabilizers and antioxidants.

29. Make-up kit according to Claim 28, **characterized in that** the additional film-forming agent is present in a content ranging from 0.5% to 19% by weight, relative to the total weight of each first and/or second composition, and preferably from 2% to 10% by weight.

30. Make-up kit according to any one of Claims 19 to 29, **characterized in that** the first and/or second composition are in the form of a nail varnish, a face powder, an eyeshadow, a foundation or a make-up product for the lips or the body.

31. Make-up kit according to any one of Claims 19 to 30, **characterized in that** it contains means for applying the first and the second compositions to keratin substances.

32. Make-up kit according to Claim 31, **characterized in that** the means are chosen from the group formed by fine and coarse brushes, feathers and sponges.

33. Make-up kit according to one of Claims 19 to 32, **characterized in that** the first and second compositions and the solid particles are packaged in separate compartments or containers.

34. Made-up support comprising a make-up which may be obtained according to the process as defined according to one of Claims 1 to 18, the support being chosen from false nails, false eyelashes, wigs, and discs or patches adhering to the skin or the lips.

35. Use, in a make-up product, of a first film-forming composition comprising a cosmetically acceptable medium and at least one first film-forming polymer, and solid particles having a substantially spherical or ovoid shape and having a density ranging from 800 kg/m³ to 10,000 kg/m³, to obtain a make-up effect with surface relief, which has good staying power.

36. Use, in a make-up product, of solid particles having a substantially spherical or ovoid shape and having a density ranging from 800 kg/m³ to 10,000 kg/m³, to obtain a make-up effect with surface relief, which has good staying power.
